**Europäisches Patentamt**

**European Patent Office**

**Office européen des brevets**

(19)

(11) Veröffentlichungsnummer : **0 358 963 B1**

(12) # EUROPÄISCHE PATENTSCHRIFT

(45) Veröffentlichungstag der Patentschrift :
**04.11.92 Patentblatt 92/45**

(51) Int. Cl.$^5$ : **A61M 5/165**

(21) Anmeldenummer : **89115051.8**

(22) Anmeldetag : **16.08.89**

(54) Filter für Infusionen und/oder Injektionen mit Entlüftung.

(30) Priorität : **24.08.88 DE 3828671**

(43) Veröffentlichungstag der Anmeldung :
**21.03.90 Patentblatt 90/12**

(45) Bekanntmachung des Hinweises auf die
Patenterteilung :
**04.11.92 Patentblatt 92/45**

(84) Benannte Vertragsstaaten :
**AT BE CH DE ES FR GB IT LI LU NL SE**

(56) Entgegenhaltungen :
**DE-A- 2 510 148**
**US-A- 4 326 957**

(73) Patentinhaber : **WEX FILTERTECHNIK GMBH**
**Industriestrasse 17 Postfach 1409**
**W-6442 Rotenburg a.d.F. (DE)**

(72) Erfinder : **Wex, Roland**
**Am Hang 28**
**W-3508 Melsungen (DE)**

(74) Vertreter : **Quermann, Helmut, Dipl.-Ing.**
**Postfach 6145 Gustav-Freytag-Strasse 25**
**W-6200 Wiesbaden (DE)**

EP 0 358 963 B1

**Beschreibung**

Die Erfindung betrifft ein Filter für Infusionen und/oder Injektionen, mit einem Filtergehäuse, einer im Filtergehäuse befindlichen Ausnehmung zur Aufnahme eines Filterelementes, Anschlußelementen zum Verbinden mit infusions- bzw. injektionsseitigen und patientenseitigen Anschlußleitungen sowie mehreren im Filtergehäuse angeordneten Entlüftungsmembranen mit diesen zugeordneten Entlüftungsöffnungen.

Derartige Filter für Infusionen und/oder Injektionen dienen dem Zweck unerwünschte Zusatzbelastungen des Organismus durch Einschwemmung von Partikeln, Mikroorganismen sowie Gas- bzw. Luftbläschen zu verhindern und damit klinische Folgeerscheinungen, wie zum Beispiel kollaterale Mikroinfarkte, Luftembolie, Sepsis usw.

Aus der Praxis ist ein Filter der genannten Art bekannt, bei dem das Filtergehäuse die Form eines Rechtecks aufweist und jeder Schmalseite des Rechtecks eine sich über deren Länge erstreckende jeweils eine Entlüftungsmembran aufweisende Entlüftungskammer mit jeweils zwei in diese eingebrachten Entlüftungsöffnungen zugeordnet ist. Die beiden Entlüftungsöffnungen sind benachbart der Mittellängsachse des Filtergehäuses positioniert, womit eine optimale Entlüftung des Filters nur dann erfolgen kann, wenn die Schmalseiten des Filtergehäuses horizontal ausgerichtet sind, da sich ansonsten die Luftbläschen in den Ecken der jeweiligen Entlüftungskammer ansammeln. Im übrigen wird es bei dem bekannten Filter als nachteilig angesehen, daß über die Gesamtlänge der Schmalseiten sich erstreckende Entlüftungsmembranen erforderlich sind, was zu einer Verringerung der wirksamen Filterfläche führt, darüber hinaus bedingt die Zuführung und Ableitung der Flüssigkeit zum bzw. vom Filter im Bereich der Entlüftungsmembranen eine aufwendige Herstellung des Filters und eine komplizierte Verbindung der beiden Filterhälften mit der Folge einer nur geringen Druckstabilität des Filters.

Aus der Praxis ist ferner ein Filter der genannten Art bekannt, bei dem jedoch oberhalb und unterhalb des Filterelementes in der jeweiligen Verbindung zu den Anschlußelementen des Filters ein großes Totvolumen zur Aufnahme von Luftbläschen und deren Ableitung durch die den Totvolumina zugeordneten Entlüftungsmembranen vorgesehen ist. Die großen Totvolumina bedingen, daß das genannte Filter für geringe Infusions- bzw. Injektionsmengen ungeeignet ist.

Aus der US-A-4.326.957 ist ein Filter der genannten Art bekannt, dessen quadratisch geformten Filter längs einer seiner Seiten eine in die Verbindungsleitung zum Infusionsbehälter mündende Lüftungsmembran zugeordnet ist.

Es ist Aufgabe vorliegender Erfindung, ein Filter der genannten Art zu schaffen, das bei einfacher baulicher Ausbildung ein sicheres Entlüften der durch das Filter tretenden Flüssigkeit gewährleistet.

Gelöst wird die Aufgabe durch ein Filter der genannten Art, das dadurch gekennzeichnet ist, daß das Filtergehäuse die Form eines Dreiecks aufweist und die Entlüftungsmembranen im Bereich der Ecken des Filtergehäuses angeordnet sind. Es ist hierdurch sichergestellt, daß unabhängig von der Position des Filters Luft oder Gase der Infusionslösung immer zu der jeweils oben stehenden Entlüftungsöffnung aufsteigen können, womit im Gegensatz zu den bekannten Filtern keine große Vorkammer zum Sammeln der Gase bzw. der Luft erforderlich ist, sondern diese vielmehr aufgrund der sich nach oben verjüngenden Innenkontur des Filtergehäuses einer der Entlüftungsöffnungen zugeführt werden und unmittelbar durch die jeweilige Entlüftungsmembran aus dem Filtergehäuse austreten können. Darüber hinaus gewährleistet die gewählte Dreieckform eine günstige Druckverteilung innerhalb des Filtergehäuses und damit eine hohe Druckbeständigkeit in jeder Art von Infusions- bzw. Injektionsbetrieb.

Vorteilhaft weist das Filtergehäuse die Form eines gleichschenkligen Dreiecks auf, womit sichergestellt ist, daß im Filtergehäuse neben dem erforderlichen Platz für die drei Entlüftungsöffnungen genügend Raum für die Anschlußelemente zum Verbinden mit den Anschlußleitungen verbleibt. Grundsätzlich sind aber beliebige Dreiecksformen für das Filtergehäuse denkbar, so insbesondere auch die Form eines gleichseitigen Dreiecks.

Gemäß einer besonderen Ausführungsform der Erfindung ist vorgesehen, daß das Filtergehäuse plattenförmig ausgebildet und mit diesem im Bereich einer Dreieckskante eine sich senkrecht zum Filtergehäuse erstreckende Auflageplatte verbunden ist. Die Auflageplatte dient zum Anbringen des Filters beispielsweise mittels eines Pflasters am Patienten, hierbei ist anzustreben, daß die Auflageplatte dauerhaft horizontal und die dieser abgewandte Entlüftungsöffnung oben zu liegen kommen, so daß die Entlüftung des Filters stets über die obere Entlüftungsöffnung und die dieser zugeordnete Entlüftungsmembran erfolgt. Ist das Filter mit seiner Auflageplatte am Patienten unmittelbar, beispielsweise in dessen liegender Position am Unterarm, befestigt, sollte sichergestellt sein, daß der Unterarm im wesentlichen horizontal ausgerichtet bleibt und damit bei einer Drehbewegung des Unterarmes um seine Längsachse die Entlüftung abwechselnd durch die jeweils oben befindlichen Entlüftungsöffnungen geschieht. Prinzipiell ist es jedoch nicht erforderlich, daß das Filtergehäuse mit einer Auflageplatte versehen ist, da eine Positionierung des Filtergehäuses im Sinne einer optimalen Entlüftung auch durch eine entsprechende Positionierung der Anschlußleitungen und damit des Filtergehäuses erzielt werden kann.

Zweckmäßig weist das Filtergehäuse in geringfügigem Abstand zu der der Auflageplatte abgewandten Entlüftungsmembran das Anschlußelement zum Verbinden mit der infusions- bzw. injektionsseitigen Anschlußleitung und in geringfügigem Abstand zu der Auflageplatte das Anschlußelement zum Verbinden mit der patientenseitigen Anschlußleitung auf. Darüber hinaus sollte die Verbindung der beiden Anschlußelemente mit dem Filtergehäuse im Bereich der durch die der Auflageplatte abgewandten Entlüftungsmembran verlaufende Winkelhalbierende der zugewandten Ecke des Dreiecks erfolgen. Es ist damit sichergestellt, daß in der anzustrebenden Betriebsstellung des Filters mit untenliegender Auflageplatte und obenliegender Entlüftungsöffnung die Flüssigkeit geringfügig unterhalb der Entlüftungsöffnung in das Filtergehäuse einströmt und an einer tieferen Stelle dieses wieder verläßt. Der höhenversetzte, untenliegende Flüssigkeitsabgang garantiert aufgrund des höherliegenden Flüssigkeitszuganges ein gutes Flüssigkeits-Durchströmungsprinzip im Filtergehäuse.

Gemäß einer besonderen Ausbildung der Erfindung ist vorgesehen, daß das Filtergehäuse aus zwei in einer Ebene senkrecht zur Auflageplatte geteilten Filterhälften besteht, wobei eine der beiden Hälften das Anschlußelement zum Verbinden mit der infusions- bzw. injektionsseitigen Anschlußleitung und die andere Hälfte das Anschlußelement zum Verbinden mit der patientenseitigen Anschlußleitung aufweist. Die beiden Filtergehäusehälften sind zweckmäßig außen miteinander verschweißt und bedingen damit die hohe Druckbeständigkeit des Filters. Innen sollte die der infusions- bzw. injektionsseitigen Anschlußleitung zugeordnete Filtergehäusehälfte im Bereich der Dreiecksseiten eine das Filterelement umgebende umlaufende Nut aufweisen. In dem von der Nut umgebenden Raum sollten die Filtergehäusehälften zweckmäßig mit Stützrippen zur Stabilisierung des Filterelementes versehen sein, insbesondere Stützrippen, die parallel zu der Winkelhalbierenden angeordnet sind, die durch die der Auflageplatte abgewandte Entlüftungsmembran verläuft. Durch die konkrete Ausgestaltung des Filters mit der oben befindlichen Entlüftungsmembran und dem geringfügig unterhalb positionierten Flüssigkeitszugang ist gewährleistet, daß die Partikel und Schwebeteilchen über die senkrechtstehenden, durch die Stützrippen gebildeten Flüssigkeitsnuten der Filterinnenseite in die untere Sammelnut absinken können. Hierdurch wird im Gegensatz zu den Filtern bekannter Art erreicht, daß die Filterfläche länger offen bleibt. Die umlaufende Nut wird aber auch als Luft- oder Gasleitnut zu den Entlüftungsmembranen genutzt. Außerhalb der umlaufenden Luft- und Gasleitnut liegt die eigentliche Filterklemmfläche und bei einer Ausbildung des Filters aus Kunststoff die Filtergehäuseschweißkontur, die zweckmäßig so ausgebildet ist, daß eine ausreichend große Menge Kunststoffmaterial plastifiziert wird, um so mindestens einen Berstdruck von sechs bar sicherzustellen.

Die dreieckige-geometrische Filtergehäuseform erlaubt die Verwendung aller möglichen Kunststoffmaterialien aus physiologisch unbedenklichem Material, das für die verschiedenen Ansprüche chemisch beständig, und für die verschiedenen Sterilisationsarten geeignet ist. Weiterhin erlaubt es die Integration verschiedenster Membran- oder Gewebematerialien, die medikamenten- oder patientennotwendig sind. Die geometrische Lösung erlaubt außerdem innerhalb des Filtergehäuses ein minimales Totvolumen, so daß auch kleinere Medikamentenmengen wirkungsvoll über dieses Filter appliziert werden können, im Gegensatz zu einer Vielzahl bekannter Lösungen.

Weitere Merkmale der Erfindung sind in der Beschreibung der Figuren und in den Unteransprüchen dargestellt, wobei bemerkt wird, daß alle Einzelmerkmale und alle Kombinationen von Einzelmerkmalen weitere erfinderische Ausgestaltungen darstellen.

In den Figuren ist die Erfindung an einer Ausführungsform beispielsweise dargestellt, ohne auf diese beschränkt zu sein. Es stellt dar:

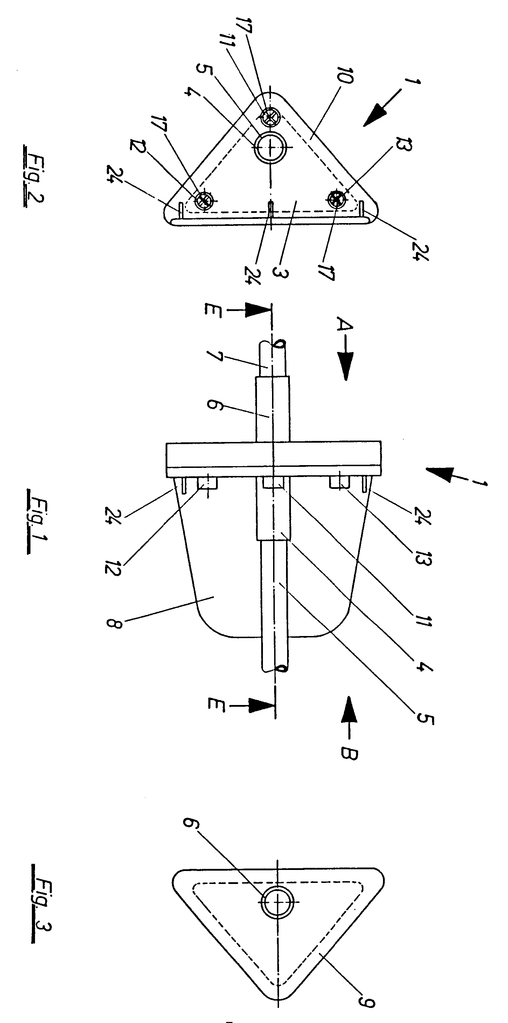

Figur 1 eine Draufsicht auf das erfindungsgemäße Filter,

Figur 2 eine Ansicht B des in Figur 1 gezeigten Filters,

Figur 3 eine Ansicht A des in Figur 1 gezeigten Filters,

Figur 4 eine Seitenansicht des Filters,

Figur 5 eine Innenansicht einer Filtergehäusehälfte in Richtung der Pfeile C in Figur 4 gesehen,

Figur 6 eine Innenansicht der anderen Filtergehäusehälfte in Richtung der Pfeile D gemäß Figur 4 gesehen und

Figur 7 einen senkrechten Schnitt durch das Filter gemäß der Linie E-E in Figur 1.

Das erfindungsgemäße Filter besteht im wesentlichen aus einem Filtergehäuse 1, einer im Filtergehäuse 1 befindlichen Ausnehmung 2 zur Aufnahme eines Filterelementes 3, einem Anschlußelement zum Verbinden mit einer infusions- bzw. injektionsseitigen Anschlußleitung 5, einem weiteren Anschlußelement 6 zum Verbinden mit einer patientenseitigen Anschlußleitung 7 sowie einer senkrecht zum Filtergehäuse 1 orientierten Auflageplatte 8.

Im Detail ist das Filtergehäuse 1 plattenförmig ausgebildet und weist, wie insbesondere in den Figuren 2, 3 und 5, 6 verdeutlicht, die Form eines Dreiecks auf. Das Filtergehäuse 1 besteht aus zwei Gehäusehälften 9 und 10, die in der Plattenebene geteilt sind. In den Ecken der Gehäusehälfte 10 sind drei Entlüftungsstutzen 11, 12 und 13 angeordnet, deren Entlüftungsöffnungen 14, 15 und 16 von hydrophoben Filtermembranen 17 bedeckt werden, die ihrerseits durch in die Entlüftungsöffnungen 14, 15 und 16 eingebrachte Stützrippenkreuze 18 abgestützt werden.

Wie der Darstellung der Figuren 2, 3 und 5, 6 zu entnehmen ist, weist das Filtergehäuse die Form eines gleichschenkligen Dreieckes auf, auf dessen Symmetrielinie die patientenseitige Filtergehäusehälfte 10 mit dem Entlüftungsstutzen 11 versehen ist. Geringfügig beabstandet von diesem ist, gleichfalls auf der Symmetrielinie 19 angeordnet das Anschlußelement 4 mit der Gehäusehälfte 10 verbunden sowie ebenfalls auf der Symmetrielinie 19 in unmittelbarer Nähe zur dem Entlüftungsstutzen 11 abgewandten Dreiecksseite 20 das Anschlußelement 6 mit der Gehäusehälfte 9 verbunden. Auf die Orientierung der Figuren 4 bis 6 bezogen befindet sich somit im obersten Bereich der Ausnehmung 2 des Filtergehäuses 1 der Entlüftungsstutzen 11, geringfügig unterhalb diesem das infusions- bzw. injektionsseitige Anschlußelement 4 und benachbart zur unteren Dreiecksseite 20 des Filtergehäuses 1 das patientenseitige Anschlußelement 6, sowie geringfügig unterhalb diesem auf gleicher Höhe die Entlüftungsstutzen 12 und 13.

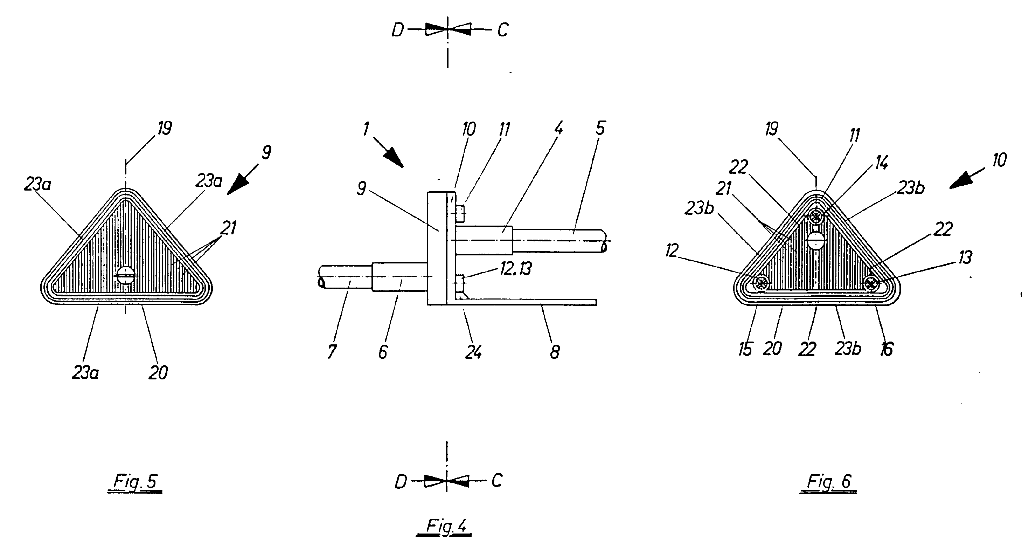

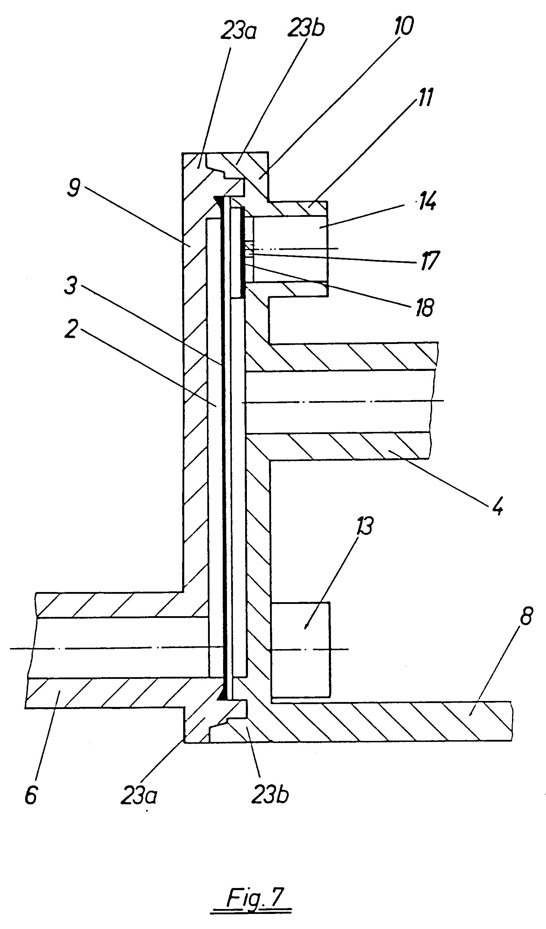

Wie der Darstellung der Figuren 5 bis 7 zu entnehmen ist, sind beide Gehäusehälften 9 und 10 innen im Bereich des Filterelementes 3 mit, auf die Orientierung nach den Figuren 5 und 6 bezogen, senkrechten Flüssigkeitsnuten 21 versehen. Die Flüssigkeitsnuten 21 der infusions- bzw. injektionsseitigen Gehäusehälfte 10 münden dabei mit ihren Enden in eine in Art eines Dreiecks diese umgebende umlaufende Nut 22. An den durch die Nut 22 begrenzten Dreiecksbereich der Gehäusehälfte 10 und den durch die Flüssigkeitsnuten 21 begrenzten Dreiecksbereich der Gehäusehälfte 9 schließt sich auf den aufeinander zugewandten Seiten der Gehäusehälften eine in Dreieckform umlaufende Filterklemmfläche 23a bzw. 23b an, wobei, wie aus der Darstellung der Figur 7 zu entnehmen ist, eine der Filterklemmflächen negativ und die andere positiv ausgebildet ist, wodurch beim Ineinanderstecken der aus Kunststoff bestehenden Gehäusehälften 9 und 10 und anschließendem Verschweißen in diesem Bereich eine ausreichend große Menge Kunststoffmaterial plastifiziert wird und mit dem so verschlossenen Filtergehäuse ein Berstdruck von sechs bar gewährleistet werden kann.

Wie der Darstellung der Figuren 4 und 7 zu entnehmen ist, stellt die Auflageplatte 8 eine Einheit mit der Gehäusehälfte 10 dar, Verstärkungsrippen 24 verstärken zusätzlich deren Verbindung. Die Auflageplatte 8 ist senkrecht zum Filtergehäuse 1 und dem Filterelement 3 orientiert und im Bereich der unteren Dreiecksseite 20 mit dem Filtergehäuse 1 verbunden.

Bei der Verwendung des Filters ist anzustreben, daß die Plattenebene der Auflageplatte 8 horizontal ausgerichtet ist und entsprechend das Filtergehäuse 1 und das Filterelement 3 vertikal mit oben befindlichem Entlüftungsstutzen 11. Dies kann beispielsweise dadurch bewerkstelligt werden, daß das Filter in einer entsprechenden Position am Bett des Patienten befestigt wird oder aber, um annäherungsweise diese Position zu gewährleisten, indem die Auflageplatte 8 entsprechend an einem im wesentlichen horizontal positionierten Unterarm des Patienten fixiert wird. In dieser Stellung fließt im Betrieb des Filters die Flüssigkeit von dem unter dem oberen Entlüftungsstutzen 11 befindlichen infusions- bzw. injektionsseitigen Anschlußelement 4 zu dem weiter unten angeordneten patientenseitigen Anschlußelement 6. Die in der Flüssigkeit befindlichen Partikel und Schwebeteilchen können dabei über die senkrechtstehenden Flüssigkeitsnuten 21 der Gehäusehälfte 10 in den unteren Bereich der Nut 22 absinken, wodurch gewährleistet wird, daß die Filterfläche des Filterelementes 3 länger offen bleibt. Die umlaufende Nut 22 wird aber auch als Luft- oder Gasleitnut zu den Entlüftungsöffnungen 14, 15 und 16 genutzt. Im übrigen fungieren die Flüssigkeitsnuten 21 beider Gehäusehälften 9 und 10 als Stützrippen für das Filterelement 3.

## Patentansprüche

1. Filter für Infusionen und/oder Injektionen, mit einem Filtergehäuse (1), einer im Filtergehäuse befindlichen Ausnehmung zur Aufnahme eines Filterelements (3), Anschlußelementen (46) zum Verbinden mit infusions- bzw. injektionsseitigen und patientenseitigen Anschlußleitungen (5, 7), sowie mehreren im Filtergehäuse angeordneten Entlüftungsmembranen (17) mit diesen zugeordneten Entlüftungsöffnungen (11, 12, 13), **dadurch gekennzeichnet,** daß das Filtergehäuse (1) die Form eines Dreiecks aufweist und die Entlüftungsmembranen (17) im Bereich der Ecken (Entlüftungsöffnungen 14, 15, 16) des Filtergehäuses (1) angeordnet sind.

2. Filter nach Anspruch 1, dadurch gekennzeichnet, daß das Filtergehäuse (1) die Form eines gleichschenkligen Dreiecks aufweist.

3. Filter nach Anspruch 1 oder 2, dadurch gekennzeichnet, daß das Filtergehäuse (1) plattenförmig ausgebildet und mit diesem im Bereich einer Dreieckskante (20) eine sich senkrecht zum Filtergehäuse (1) erstreckende Auflageplatte (8) verbunden ist.

4. Filter nach einem der Ansprüche 1 bis 3, dadurch gekennzeichnet, daß das Filtergehäuse (1) in geringfügigem Abstand zu der der Auflageplatte (8) abgewandten Entlüftungsmembran (17) das Anschlußelement (4) zum Verbinden mit der infusions- bzw. injektionsseitigen Anschlußleitung (5) und in geringfügigem Abstand zu der Auflageplatte (8) das Anschlußelement (6) zum Verbinden mit der patientenseitigen Anschlußleitung (7) aufweist.

5. Filter nach Anspruch 4, dadurch gekennzeichnet, daß die Verbindung der beiden Anschlußelemente (4, 6) mit dem Filtergehäuse (1) im Bereich der durch die der Auflageplatte (8) abgewandten Entlüftungsmembran (17) verlaufende Winkelhalbierende (19) der zugewandten Ecke des Dreiecks erfolgt.

6. Filter nach Ansprüch 4 oder 5, dadurch gekennzeichnet, daß das Filtergehäuse (1) aus zwei in einer Ebene senkrecht zur Auflageplatte (8) geteilten Filterhälften (9, 10) besteht, wobei eine der beiden Hälften (10) das Anschlußelement (4) zum Verbinden mit der infusions- bzw. injektionsseitigen Anschlußleitung (5) und die andere Hälfte (9) das Anschlußelement (6) zum Verbinden mit der patientenseitigen Anschlußleitung (7) aufweist.

7. Filter nach Anspruch 6, dadurch gekennzeichnet, daß die beiden Filtergehäusehälften (9, 10) miteinander verschweißt sind.

8. Filter nach Anspruch 6 oder 7, dadurch gekennzeichnet, daß die dem infusions- bzw. injektionsseitigen Anschlußelement (4) zugeordnete Filtergehäusehälfte (10) im Bereich der Dreiecksseiten innen eine das Filterelement (3) umgebende umlaufende Nut (20) aufweist.

9. Filter nach einem der Ansprüche 1 bis 8, dadurch gekennzeichnet, daß die Filtergehäusehälften (9, 10) Stützrippen (21) für das Filterelement (3) aufweisen, insbesondere Stützrippen (21), die parallel zu der Winkelhalbierenden (19) angeordnet sind, die durch die der Auflageplatte (8) abgewandte Entlüftungsmembran (17) verläuft.

10. Filter nach einem der Ansprüche 1 bis 9, dadurch gekennzeichnet, daß jede Entlüftungsmembran (17) mit einem Stützelement (17), insbesondere einem Stützrippenkreuz (17) versehen ist.

11. Filter nach einem der Ansprüche 1 bis 10, gekennzeichnet durch ein aus Kunststoff bestehendes Filter.

## Claims

1. Filter for infusions and/or injections, with a filter housing (1), a recess for accommodating a filter element (3) and situated in the filter housing, connecting elements (4, 6) for connection to connecting conduits (5, 7) arranged at the infusion or injection side and at the patient's side respectively, and also a plurality of venting membranes (17) which have venting apertures (11, 12, 13) associated therewith and which are arranged in the filter housing, characterised in that the filter housing (1) is in the form of a triangle, and the venting membranes (17) are situated in the region of the corners (venting apertures 14, 15, 16) of the filter housing (1).

2. Filter according to claim 1, characterised in that the filter housing (1) is in the form of an isosceles triangle.

3. Filter according to claim 1 or 2, characterised in that the filter housing (1) is made plate-shaped, and to it there is connected, in the region of a triangle edge (20), a supporting-surface plate (8) which is disposed at right angles to the filter housing (1).

4. Filter according to one of claims 1 to 3, characterised in that the filter housing (1) comprises, at a slight spacing from the venting membrane (17) remote from the supporting-surface plate (8), the connecting element (4) for connection to the connecting conduit (5) at the infusion or injection side, and, at a slight spacing from the supporting-surface plate (8), the connecting element (6) for connection to the patient-side connecting conduit (7).

5. Filter according to claim 4, characterised in that the two connecting elements (4, 6) are connected to the filter housing (1) in the region of the bisector (19) of the angle at the facing corner of the triangle, which bisector extends through the venting membrane (17) remote from the supporting-surface plate (8).

6. Filter according to claim 4 or 5, characterised in that the filter housing (1) comprises two filter housing halves (9, 10) divided in a plane at right angles to the supporting-surface plate (8), and one of the two halves (10) has the connecting element (4) for connecting to the connecting conduit (5) situated at the infusion or injection side, and the other half (9) has the connecting element (6) for connection to the patient-side connecting conduit (7).

7. Filter according to claim 6, characterised in that the two filter housing halves (9, 10) are welded to one another.

8. Filter according to claim 6 or 7, characterised in that the filter housing half (10) associated with the connecting element (4) situated at the infusion or injection side has, in the region of the triangle sides, internally an encircling groove (20) surrounding the filter element (3).

**9.** Filter according to one of claims 1 to 8, characterised in that the filter housing halves (9, 10) have supporting ribs (21) for the filter element (3), especially supporting ribs (21) which are situated parallel to the angle bisector (19) which extends through the venting membrane (17) remote from the supporting-surface plate (8).

**10.** Filter according to one of claims 1 to 9, characterised in that each venting membrane (17) is provided with a supporting element (17), especially a supporting rib cross (17).

**11.** Filter according to one of claims 1 to 10, characterised by a filter made of synthetic plastic material.

**Revendications**

1) Filtre pour infusions et/ou injections, avec un boîtier (1), une partie en creux se trouvant dans le boîtier de filtre destinée à recevoir un élément filtrant (3), des éléments de raccordement (4,6) pour assurer l'emboîtement de conduits de liaison (5,7) entre le côté d'infusion ou d'injection et le côté du patient, ainsi que plusieurs membranes (17) de purge d'air disposées dans le boîtier du filtre avec des ouvertures (11,12,13) de désaération associées à celles-ci, filtre caractérisé en ce que le boîtier de filtre (1) a une forme triangulaire et les membranes de purge d'air (17) sont disposées dans les zones de coin (ouvertures de désaération 14,15,16) du boîtier de filtre (1).

2) Filtre selon la revendication 1, caractérisé en ce que le boîtier de filtre à la forme d'un triangle équilatéral.

3) Filtre selon la revendication 1 ou la revendication 2, caractérisé en ce que le boîtier de filtre (1) a une forme de plaque et une plaque d'appui (8) s'étendant perpendiculairement au boîtier de filtre (1) se trouve reliée à celui-ci au niveau d'une arête (20) du triangle.

4) Filtre selon une des revendications 1 à 3, caractérisé en ce que le boîtier de filtre (1) comporte à une faible distance de la membrane de purge d'air (17) opposée à la plaque d'appui (8), l'élément de raccordement (4) destiné à l'emboîtement du conduit de liaison (5) du côté de l'infusion ou injection et à une faible distance de la plaque d'appui (8) l'élément de raccordement (6) destiné à emboîter le conduit de liaison du côté patient.

5) Filtre selon la revendication 4, caractérisé en ce que la réunion des deux éléments de raccordement (4,6) avec le boîtier de filtre (1) a lieu au niveau de la bissectrice (19) de l'angle tourné vers le triangle, bissectrice traversant la membrane (17) de purge d'air opposée à la plaque d'appui (8).

6) Filtre selon la revendication 4 ou la revendication 5, caractérisé en ce que le boîtier de filtre (1) est composé de deux moitiés de filtre (9,10) séparées dans un plan perpendiculaire à la plaque d'appui (8), une des deux moitiés (10) comportant l'élément de raccordement (4) destiné à l'emboîtement avec le conduit de liaison (5) du côté de l'infusion ou injection et l'autre moitié (9) comportant l'élément de raccordement (6) destiné à l'emboîtement avec le conduit de liaison (7) du côté patient.

7) Filtre selon la revendication 6, caractérisé en ce que les deux moitiés de boîtier de filtre (9,10) sont réunies ensemble par soudage.

8) Filtre selon la revendication 6, caractérisé en ce que la moitié de boîtier de filtre (10) associée à l'élément de raccordement (4) du côté de l'infusion ou injection comporte intérieurement dans la zone des côtés du triangle une rainure (20) circulante entourant l'élément de filtre (3).

9) Filtre selon une des revendications 1 à 8;, caractérisé en ce que les moitiés de filtre (9,10) comportent des nervures d'appui (21) pour l'élément de filtre (3), en particulier des nervures d'appui (21), qui sont disposées parallèlement à la bissectrice d'angle (19), qui traverse la membrane (17) de purge d'air opposée à la plaque d'appui (8).

10) Filtre selon une des revendications 1 à 9, caractérisé en ce que chaque membrane (17) de purge d'air est munie d'un élément d'appui (17), constitué en particulier de nervures d'appui (17) en croix.

11) Filtre selon une des revendications 1 à 10, caractérisé par un filtre en matière plastique.

Fig. 2

Fig. 1

Fig. 3

Fig. 4

Fig. 5

Fig. 6

Fig. 7